# FASCICULE DE BREVET EUROPEEN

(11) **EP 3 481 442 B1**
(45) Date de publication et mention de la délivrance du brevet: **19.08.2020**
(21) Numéro de dépôt: 17748528.1
(22) Date de dépôt: 10.07.2017
(51) Int. Cl.: B05B 17/06, B05B 7/00, A61L 9/14, A61L 2/22

(54) **DISPOSITIF DE NEBULISATION D'UN PRODUIT LIQUIDE, PROCÉDÉ POUR LA DESINFECTION PAR VOIE AERIENNE ET UTILISATION DE CE DISPOSITIF POUR LA DÉSINFECTION PAR VOIE AÉRIENNE**
VORRICHTUNG ZUR VERNEBELUNG EINER FLÜSSIGKEIT, VERFAHREN ZUR DESINFEKTION DURCH LUFTWEGE UND VERWENDUNG DIESER VORRICHTUNG ZUR DESINFEKTION DURCH LUFTWEGE
DEVICE FOR NEBULIZING A LIQUID, PROCESS FOR AIRBORNE DISINFECTING AND USE OF THIS DEVICE FOR AIRBORNE DISINFECTING

(30) Priorité: 11.07.2016 FR 1656637
(43) Date de publication de la demande: 15.05.2019
(73) Titulaire: Ecolab USA, Inc., St. Paul, Minnesota 55102 (US)
(72) Inventeur: DECOSTER, Thomas, 59260 HELLEMMES-LILLE (FR); BURET, Jérémie, 59260 HELLEMMES-LILLE (FR)
(74) Mandataire: Plasseraud IP
(86) Numéro de dépôt international: PCT/FR2017/051882
(87) Numéro de publication internationale: WO 2018/011501

(56) Documents cités:
- WO-A1-2016/001492
- WO-A1-2016/096818
- DE-A1- 2 239 950
- JP-B2- 4 037 918
- US-A- 5 224 651

## Description

L'invention concerne un dispositif de nébulisation d'un produit liquide, convenant pour la désinfection de locaux par voie aérienne, ainsi qu'un procédé pour la désinfection d'un local par voie aérienne mis en œuvre par un tel dispositif.

Le domaine de l'invention est celui de la désinfection par voie aérienne (acronyme D.V.A) en particulier de local, et plus encore des dispositifs de nébulisation utilisés pour la mise en œuvre de telles désinfections.

L'invention peut également être employée pour la désinfection par voie aérienne d'un compartiment ou d'un habitacle d'un véhicule, notamment d'un véhicule de secours tel qu'une ambulance.

On sait que des agents infectieux, tels que des bactéries, virus et champignons sont en suspension dans l'air, mais aussi présents sur les objets et les parois d'un local. Les agents infectieux en suspension dans l'air peuvent se sédimenter sur les objets et les parois du local et ceux déjà présents sur les objets et les parois peuvent à nouveau être en suspension dans l'atmosphère.

Dans le cadre de la désinfection de locaux nécessitant un niveau de désinfection très élevé, tels que des blocs opératoires ou salles blanches, il est donc crucial de non seulement désinfecter les objets et les sols du local, mais aussi les parois latérales ainsi que d'inactiver les agents infectieux en suspension dans l'air. Des telles exigences de désinfection de locaux peuvent également être rencontrées dans des véhicules mobiles tels que des ambulances, ou encore, par exemple, dans certains locaux de l'industrie agroalimentaire afin d'éviter la contamination des produits alimentaires.

A la connaissance de la Demanderesse, la technique de nébulisation couramment utilisée pour pulvériser un liquide désinfectant à la granulométrie souhaitée repose sur l'utilisation de système de buse fonctionnant sur le principe d'un venturi et nécessitant une source d'air comprimée : les dispositifs de nébulisation utilisant cette technique requièrent ainsi la présence d'un compresseur d'air qui est un composant très coûteux du dispositif, énergivore et bruyant, et lourd. Selon les constatations de l'inventeur, les compresseurs économiquement disponibles pour cette application présentent pour défaut d'utiliser des moteurs électriques à balais, qui sont des composants à usure importante. De manière notable également, un compresseur à air est un équipement générant des perturbations électro-magnétiques importantes, ce qui est à éviter en particulier en milieu hospitalier.

Le document FR 2972357 est un exemple d'un tel dispositif.

On connait encore de la littérature du domaine de la désinfection par voie aérienne les techniques de nébulisation couramment appelée *«fontaine brumisante* ». Cette technique de brumisation consiste à soumettre directement un liquide contenu dans un réservoir (ouvert) à des ondes ultrasoniques, ce qui génère des cavitations et ainsi un brouillard à la surface du liquide dans ce réservoir : le brouillard désinfectant est entrainé grâce à un flux d'air généré par un système de ventilation. Les documents FR 2655279 ou encore FR 2941378 sont des exemples de tels dispositifs de nébulisation utilisant cette technique de génération de brouillard désinfectant. Selon les constatations des inventeurs, une telle technique de nébulisation pour laquelle le liquide contenu dans un réservoir est soumis directement à des ultrasons présente toutefois de nombreux inconvénients, tels que :
- l'utilisation de transducteurs piézoélectriques en immersion dans le liquide désinfectant du réservoir,
- une recirculation du produit désinfectant en ce que le brouillard généré se dépose au moins en partie sur les parois latérales du réservoir (ou d'une cheminée en aval, voire sur la « *buse d'injection* » du document FR 2941378), les gouttelettes retombant ensuite dans le réservoir,
- un débit de brouillard en sortie non maitrisé/maitrisable,
- des risques de déversement du produit désinfectant contenu dans le réservoir en cas de renversement du dispositif.

Plus particulièrement, et selon les constatations de l'inventeur, l'utilisation de transducteur piézoélectrique en immersion dans un liquide désinfectant, corrosif par nature, réduit considérablement la durée de vie du transducteur, et donc du dispositif.

Le recyclage de produit désinfectant par dépôt du brouillard et la rechute du produit déposé dans le réservoir nuit à la stabilité du produit désinfectant. Selon les observations de l'inventeur, le produit ainsi recyclé subit plusieurs phases de cavitation qui peuvent altérer le produit chimique désinfectant au niveau de ses molécules, et au final l'efficacité de la désinfection.

Les risques de déversement représentent un problème de sécurité pour l'utilisateur par contact du produit désinfectant avec la peau, et risque de brulure associé. Pour toutes ces raisons, et selon les constatations de l'inventeur, une telle technique de nébulisation n'a pas rencontré le succès escompté dans le domaine de désinfection par voie aérienne.

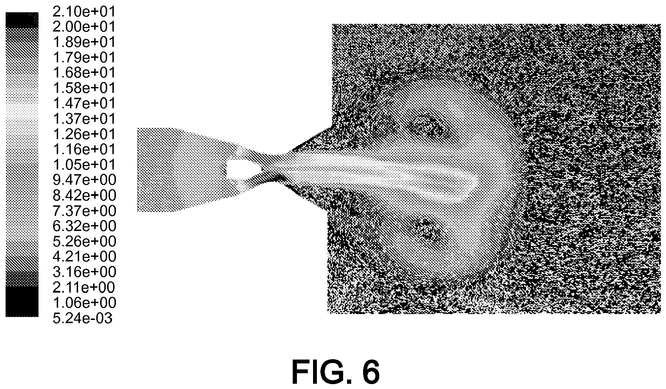

On connait encore du domaine de la pulvérisation en extérieur le dispositif du document US 5 224 651 et qui combine :
- un ventilateur, électrique, coaxial, créant un flux d'air entre les parois d'une tuyère extérieure et d'une tuyère intérieure, coaxiale l'une à l'autre, guidant le flux d'air pulsé entre les parois concentriques des tuyères,
- un dispositif de pulvérisation à buse ultrasonique agencé coaxialement aux tuyères, logé dans la tuyère intérieure et assurant la pulvérisation du liquide grâce à une buse ultra-sonique selon la direction axiale des tuyères,
- un compresseur d'air dont l'entrée d'air encercle la sortie de la buse ultrasonique, le compresseur canalisant l'air aspiré dans un inter-espace annulaire défini entre la tuyère inférieure et le compresseur, et alimentant l'air compressé par une ouverture annulaire, encerclant la sortie de la buse ultrasonique, cette ouverture annulaire présentant des déflecteurs, illustrés à la figure 6 du document, assurant une mise en rotation du flux.

Selon la description du document US 5 224 651, un tel dispositif consomme une puissance de 1000 à 1200 Watts, juste pour son compresseur : il s'agit d'un dispositif adapté à la pulvérisation en extérieur, utilisé dans le domaine de l'agriculture notamment, mais inadapté à la désinfection par voie aérienne de locaux.

On connaît encore du domaine général des dispositifs pulvérisateurs à ultrasons, le dispositif du document FR 2 285 930 comprenant :
- une tête ultrasonique comprenant un transducteur piézoélectrique, et une sonotrode conique, transmettant les vibrations du transducteur, à partir de la base de la sonotrode en contact avec le transducteur jusqu'à un plateau de pulvérisation à l'extrémité distale de la sonotrode, cette dernière comprenant un canal d'amené du liquide à pulvériser jusqu'audit plateau de pulvérisation,
- une pompe et un tube d'alimentation acheminant un liquide puisé dans un réservoir jusqu'au canal d'amenée de la sonotrode,

Selon cette antériorité, un système aéraulique crée un flux d'air d'entraînement des particules pulvérisées par la tête ultrasonique, ce système comprenant une tuyère et un ventilateur soufflant dans la tuyère, cette dernière canalisant le flux d'air jusqu'à un orifice d'échappement.

De manière notable, la tête ultrasonique est agencée interne à la tuyère, coaxialement à cette dernière, c'est-à-dire l'axe de la sonotrode confondu avec celui de la tuyère. De manière notable, le plateau pulvérisateur est placé à l'intérieur de la tuyère et la tuyère présente un rétrécissement en direction de son orifice d'échappement formant un étroit passage annulaire ménagé entre le bord de l'orifice et celui du plateau de pulvérisation : cette section réduite de passage assure un courant d'air de vitesse d'écoulement très élevée, à proximité immédiate du plateau de pulvérisation: l'effet recherché dans ce dispositif est ici d'entrainer la totalité du brouillard en évitant la formation de filets de liquide sur le plateau, et comme expliqué dans cette antériorité. De manière notable également, la sonotrode est précédée dans la tuyère par un carter intérieur qui reçoit l'électronique de commande du transducteur.

On connaît encore du domaine des dispositifs de pulvérisation de produits cosmétiques les dispositifs des documents EP 2 090 187 A1 ou EP 2 090 370 A1, qui comme le document précédent, repose sur l'utilisation d'une tête ultrasonique comprenant un transducteur piézoélectrique, et une sonotrode conique, ainsi qu'un système aéraulique configuré pour créer un flux d'air d'entraînement des particules pulvérisées par la tête ultrasonique.

Comme le document précédent, la tête ultrasonique des dispositifs des documents EP 2 090 187 A1 ou EP 2 090 370 A1 est sensiblement coaxiale à l'axe de la tuyère. De la même manière, on assure l'entrainement des particules pulvérisées par le plateau de la sonotrode grâce à un rétrécissement de tuyère en direction de son orifice d'échappement, un étroit passage annulaire étant ménagé entre le bord dudit orifice et celui du plateau de pulvérisation.

Selon les constatations de l'inventeur, de tels dispositifs de pulvérisation selon les documents FR 2285 930, EP 2090 187 A1 ou EP 2090370 A1 trouvent des applications dans le domaine de la cosmétique, en particulier l'application de produits coiffants sur des cheveux. En revanche, et à la connaissance de la Demanderesse de tels dispositifs ne trouvent pas d'application dans le domaine de la désinfection par voie aérienne, pour la création d'un brouillard désinfectant.

Selon les constatations de l'inventeur, les dispositifs à rétrécissement de section de ces antériorités génèrent de fortes turbulences qui ne permettent pas de garantir une bonne maîtrise et stabilité de la granulométrie du brouillard généré par la tête ultrasonique.

Or l'homme du métier du domaine de la désinfection par voie aérienne sait que la qualité du brouillard, en particulier la qualité de sa granulométrie est essentielle dans le succès de la désinfection d'un local, cette granulométrie devant être ni trop petite, ni trop grosse, et d'écart type le plus petit possible.

On connaît également du document FR 3 011 623 A1 un dispositif destiné à traiter de l'air devant alimenter l'enceinte d'un système, notamment un habitacle de véhicule, mais également une pièce ou salle d'un bâtiment.

Le traitement de l'air réalisé par le dispositif du document FR 3 011 623 A1 consiste essentiellement en un rafraîchissement de l'enceinte par adjonction de (très) petites gouttes de liquide, ce liquide pouvant éventuellement être de l'eau, éventuellement additionné d'un désinfectant destiné à assainir l'enceinte.

Le dispositif de traitement de l'air selon le mode de réalisation de la figure 3 du document FR 3 011 623 A1, comporte à cet effet des moyens de production agencés de manière à produire pour une enceinte un flux d'air traité par adjonction de petites gouttes de liquide au moyen d'une technique de nébulisation utilisant au moins un oscillateur piézoélectrique couplé à une chambre de nébulisation, ledit oscillateur piézoélectrique faisant partie d'un élément à membrane perforée, alimenté en liquide par un tuyau, permettant de générer de très petites gouttes au niveau d'une sortie de l'élément, destinées à être mélangées à un flux d'air à traiter circulant dans un conduit.

La sortie de l'élément piézoélectrique est située au niveau de la sortie du conduit afin que les gouttes soient mélangées au flux d'air dans l'enceinte.

L'élément piézoélectrique est solidarisé au plafonnier de l'habitacle et la sortie de l'élément piézoélectrique est inclinée par rapport à la sortie du conduit, afin de délivrer de façon optimale les gouttes dans le flux d'air en aval de la sortie du conduit.

Il est également envisagé dans le document FR 3 011 623 A1 que la sortie de l'élément piézoélectrique débouche dans le conduit en amont de la sortie du conduit, de préférence au voisinage de la sortie du conduit.

Le dispositif décrit dans le document FR 3 011 623 A1 présente l'inconvénient qu'il ne peut pas être employé pour la désinfection par voie aérienne, qui nécessite l'emploi de produits désinfectants tels que le peroxyde d'hydrogène, l'acide péracétique ou l'acide acétique.

En effet, la membrane souple perforée du dispositif de nébulisation du document FR 3 011 623 A1 n'est pas adaptée pour assurer la nébulisation de tels produits, en ce qu'elle risque de se détériorer rapidement au contact de ces derniers.

De plus, pour réaliser la désinfection par voie aérienne de locaux ou de compartiments de véhicule, les quantités de ces produits permettant d'atteindre le seuil de désinfection dépassent sensiblement les seuils limites d'exposition pour l'Homme. Or, le dispositif décrit dans le document FR 3 011 623 A1 est prévu pour fonctionner dans un habitacle de véhicule en présence d'au moins un occupant et n'est donc pas adapté pour la désinfection par voie aérienne.

On connaît également du document DE 22 39 950 A1 un dispositif portatif pour l'atomisation d'un liquide, notamment de produits cosmétiques, de produits pour l'extermination des insectes ou l'amélioration de la qualité de l'air.

Comme visible sur l'exemple de réalisation de la figure 1 du document DE 22 39 950 A1, un tel dispositif portatif comporte un boitier dans lequel se trouve un ventilateur entraîné par un moteur. Un système d'oscillation piézoélectrique est disposé dans le flux d'air généré par le ventilateur, à l'intérieur des parois du boîtier.

Le système d'oscillation piézoélectrique comporte une sonotrode en tronc de cône, sur laquelle est fixé un convertisseur piézoélectrique, sous la forme d'un disque en céramique piézoélectrique. Sur l'extrémité de plus faible section du tronc de cône se trouve la plaque de travail, à la surface de laquelle un liquide est atomisé en un brouillard de très fines gouttelettes par l'intermédiaire des oscillations ultrasoniques du système.

Le liquide est pulvérisé à la surface de la plaque de travail par l'intermédiaire d'une buse de pulvérisation, depuis un réservoir par l'intermédiaire d'un dispositif de convoyage et à travers un tuyau d'alimentation.

Afin de souffler le brouillard, la plaque forme un angle faible, par exemple, par rapport au flux d'air. Grâce au flux une importante accumulation de liquide à la surface de la plaque de travail est déjà en grande partie empêchée.

Le dispositif du document DE 22 39 950 A1 présente l'inconvénient que le système d'oscillation piézoélectrique ainsi que la buse de pulvérisation, qui n'est pas intégrée au système d'oscillation piézoélectrique, se trouvent intégralement dans le boîtier afin de pouvoir coopérer avec le flux d'air généré par le ventilateur.

Ainsi, le flux d'air en sortie du dispositif sera fortement perturbé par la présence de ces différents éléments dans le boîtier et le contrôle du débit et de la granulométrie du brouillard pulvérisé en sera fortement complexifié.

On connaît également du document US 2010/044460 A1 un atomiseur de fluide permettant de diffuser un liquide sous forme de microgouttelettes par l'intermédiaire de moyens piézoélectriques, employé notamment pour la désinfection ou l'humidification de l'air.

Comme visible sur la figure 15 du document US 2010/044460 A1, l'atomiseur comporte un corps de transducteur (piézoélectrique) en acier inoxydable, une cavité interne contenant le liquide. Une rondelle de matériau céramique piézoélectrique est prévue, reliée à une électrode pour alimenter en électricité le matériau piézoélectrique qui permettra de faire vibrer une membrane percée afin d'obtenir des microgouttelettes.

A une fréquence de 70 kHz, il est possible d'obtenir avec ce dispositif des gouttelettes d'un diamètre de 2 µm s'écoulant à un débit de 2,5 ml/min.

Comme visible sur la figure 16B du document US 2010/0044460 A1, ce document décrit également un inhaleur pour l'administration de médicaments dans les poumons d'un patient.

Un tel inhaleur comporte un adaptateur pour la bouche associé à un module en forme de T, dans lequel est incorporé l'atomiseur décrit précédemment. Lorsque l'atomiseur est en marche, celui-ci génère un aérosol à l'intérieur du module et le patient inhale cet aérosol par l'intermédiaire de l'adaptateur pour la bouche.

Un tel dispositif présente plusieurs inconvénients. En effet, l'emploi d'une membrane pour réaliser l'atomisation du liquide ne permet pas d'employer le dispositif du document US 2010/0044460 A1 pour faire de la DVA de locaux ou de compartiments de véhicules, en ce que les produits employés pour effectuer la DVA, dans les quantités efficaces pour désinfecter des espaces volumineux comme des locaux ou des habitacles de véhicule, risqueraient de détériorer la membrane rapidement.

De plus, comme visible sur la figure 16 du document US 2010/0044460 A1, l'atomiseur est placé sensiblement au milieu de l'inhaleur, à une certaine distance de la sortie, matérialisée par l'adaptateur pour la bouche. Ainsi, des gouttes de liquide risquent de se déposer sur les parois de l'inhaleur entre l'atomiseur et la sortie, ce qui va générer un recyclage du produit au cours de l'utilisation de l'inhaleur et donc altérer le produit, mais également nuire à la maîtrise du débit et de la granulométrie du liquide atomisé en sortie de l'inhaleur.

On connaît également du document US 2009/224066 A1 un dispositif de pulvérisation pour réaliser des revêtements fins sur les aliments ou sur les emballages pour aliments, prévu pour être utilisé dans l'industrie alimentaire, donc inadapté pour la DVA, en particulier de locaux ou de compartiments de véhicules.

Un tel dispositif peut par exemple être employé pour pulvériser une solution antimicrobienne ou de brunissement anti-enzymatique afin de servir de revêtement.

Comme visible sur l'exemple de réalisation de la figure 1 du document US 2009/224066 A1, le dispositif de pulvérisation comporte un bec à ultrasons connecté à une alimentation en liquide, elle-même connectée à un réservoir.

Le bec à ultrasons comporte une corne avant configurée pour fonctionner comme une section d'atomisation. La corne avant est configurée pour former les gouttes de liquide introduites dans le bec à ultrasons.

Le bec à ultrasons comporte également une portion de transducteur qui inclut une paire de transducteurs positionnés dans une section intermédiaire du bec à ultrasons. Ce sont des transducteurs piézoélectriques configurés pour transmettre le mouvement mécanique à fréquences ultrasoniques à la corne avant permettant à la surface d'atomisation disposée sur la corne avant de vibrer à une fréquence ultrasonique avec une amplitude suffisante permettant l'atomisation du liquide.

Comme visible sur l'exemple de réalisation de la figure 4 du document US 2009/224066 A1, un tel dispositif de pulvérisation comporte un embout déflecteur à jet d'air plat positionné de façon adjacente à une surface d'atomisation d'un bec à ultrasons, les deux étant disposés sur un ensemble de bloc de jets. Un raccord d'alimentation d'air et un raccord d'alimentation en liquide sont également prévus sur l'ensemble de bloc de jets.

En fonctionnement, une source d'air est attachée au raccord d'alimentation d'air et un liquide à atomiser est connecté au raccord d'alimentation en liquide. Le flux d'air du raccord d'alimentation d'air est envoyé à travers l'embout déflecteur à jet d'air, pour lui donner une forme aplatie. L'air aplati subit une déflection par la surface d'atomisation du bec à ultrasons. Le liquide ayant pénétré par l'intermédiaire du raccord d'alimentation en liquide est atomisé par le bec à ultrasons et est éjecté au niveau de la surface d'atomisation. Le liquide atomisé est entraîné dans le flux d'air aplati produisant un motif tourbillonnant qui est composé d'air et du liquide atomisé.

Ainsi, un tel dispositif présente l'inconvénient que le brouillard de liquide, en sortie du dispositif de pulvérisation, sera très fortement perturbé par son interaction avec le bec à ultrasons. De plus, le flux d'air n'étant pas canalisé dans un conduit fermé, celui-ci sera également fortement perturbé. Il s'avère donc très compliqué de maîtriser la granulométrie du brouillard ou son débit en sortie d'un tel dispositif de pulvérisation.

Le but de la présente invention est de pallier les inconvénients précités en proposant un dispositif de nébulisation convenant pour la génération d'un brouillard désinfectant dont la qualité de la granulométrie permet d'obtenir de bonnes performances de désinfection, de coût de revient maitrisé, ne requérant pas de compresseur à air pour sa mise en œuvre, faiblement énergivore.

Plus encore, le but de la présente invention est de proposer un tel dispositif de nébulisation permettant un contrôle précis du débit du brouillard désinfectant pulvérisé. Un autre but de la présente invention est de proposer un tel dispositif de pulvérisation assurant une pulvérisation, avantageusement sans recyclage de produit, et ainsi sans risque d'altérer le produit désinfectant.

Un autre but de la présente invention est de proposer un tel dispositif de pulvérisation de sécurité accrue, évitant les risques de déversement de produits en cas de renversement du dispositif.

Un autre but de la présente invention est de proposer un tel dispositif portatif compact et léger.

Un autre but de la présente invention est de proposer un procédé pour la désinfection par voie aérienne mis en œuvre au moyen d'un dispositif de nébulisation.

D'autres buts et avantages de la présente invention apparaitront au cours de la description qui va suivre qui n'est donnée qu'à titre indicatif et qui n'a pas pour but de la limiter.

Aussi, l'invention concerne un dispositif de nébulisation d'un produit convenant pour la désinfection par voie aérienne, comprenant :
- une tête ultrasonique comprenant un transducteur et une sonotrode configurée pour transmettre les vibrations depuis le transducteur vers une surface d'éjection de particules, la sonotrode comportant un canal d'amenée du produit à pulvériser vers la surface d'éjection, la sonotrode présentant un corps de révolution terminé par un plateau formant ladite surface d'éjection, l'axe de la sonotrode définissant l'axe de la tête ultrasonique,
- un système aéraulique configuré pour créer un flux d'air d'entraînement des particules pulvérisées par la tête ultrasonique, comprenant une tuyère canalisant le flux d'air jusqu'à une sortie d'air.

Selon l'invention, la tête ultrasonique est agencée au niveau de la sortie d'air de manière à positionner dans le flux d'air d'entraînement la surface d'éjection et une partie de longueur de la sonotrode à partir de ladite surface d'éjection tandis qu'une partie du corps de la tête ultrasonique fait saillie radialement extérieurement au-delà du diamètre de l'orifice de sortie d'air de la tuyère et de manière à ne pas être soumis audit flux d'air d'entraînement.

De plus, l'axe de la tête ultrasonique est agencé sensiblement perpendiculaire à l'axe de la tuyère, la surface d'éjection étant sensiblement parallèle à l'axe de la tuyère .

Selon des caractéristiques de l'invention, prises seules ou en combinaison :
- la tête ultrasonique présente un carter, en particulier cylindrique, recouvrant le transducteur, voire une électronique de commande du transducteur piézoélectrique, ainsi qu'une partie proximale de la sonotrode, une partie distale de la sonotrode portant la surface d'éjection s'étendant axialement au-delà du carter et dans lequel la tête ultrasonique est agencée de manière à positionner dans le flux d'entraînement seulement la surface d'éjection et la partie distale de la sonotrode et de sorte que le carter soit positionné extérieurement en saillie au-delà du diamètre de l'orifice de sortie d'air pour ne pas être soumis audit flux d'air d'entraînement ;
- la partie de la tête ultrasonique soumise au flux d'entraînement est positionnée en aval de l'orifice de sortie d'air de la tuyère, selon le sens d'écoulement du flux d'air ;
- la partie de la tête ultrasonique soumise au flux d'entraînement est positionnée à proximité immédiate de l'orifice de sortie d'air de la tuyère ;
- la tuyère est de section constante en longueur, au moins au niveau de la sortie d'air de tuyère, voire sur toute sa hauteur ;
- la tuyère est constituée essentiellement par un corps cylindrique dont la base à l'embouchure inférieure constitue une entrée pour la tuyère, et la base à l'embouchure supérieure ladite sortie d'air ;
- ledit système aéraulique comprend un moyen électrique de ventilation, disposé en partie inférieure de la tuyère ;
- ledit moyen de ventilation électrique est un ventilateur positionné en partie basse de la tuyère dont l'axe de rotation de l'hélice est sensiblement coaxial à l'axe de la tuyère ;
- un système d'aubage est agencé entre ladite sortie d'air et le moyen électrique de ventilation, configuré pour redresser le flux généré par ledit moyen électrique de ventilation ;
- une pompe péristaltique est configurée pour acheminer le produit à pulvériser jusqu'au canal d'amenée de la tête ultrasonique ;
- le dispositif est portatif et comporte une surface d'appui permettant de positionner la sortie d'air de la tuyère vers le haut, lorsque posée sur une surface horizontale ;
- l'axe de la tuyère est sensiblement à la verticale, ou suivant une direction inclinée par rapport à la verticale d'un angle α compris entre 0° et 60°, tel que par exemple 45° lorsque la surface d'appui repose sur la surface horizontale ;
- le dispositif comprend un boîtier recevant intérieurement une électronique de contrôle, ladite surface inférieure dudit boitier constituant ladite surface d'appui, ledit système aéraulique étant agencé solidaire du boitier, positionné en porte à faux par rapport à la surface d'appui du boitier, de manière à positionner ladite entrée d'air inférieure du système aéraulique à distance Δ de la surface horizontale.

L'invention concerne encore un procédé pour la désinfection par voie aérienne mis en œuvre au moyen d'un dispositif de nébulisation selon l'invention, dans lequel :
- on génère un brouillard désinfectant de débit déterminé par l'action de la surface d'éjection de la tête ultrasonique en alimentant en produit désinfectant le canal d'amenée selon le débit déterminé,
- on entraîne le brouillard désinfectant de débit déterminé dans le flux d'air généré à la sortie d'air de la tuyère.

Selon des caractéristiques optionnelles du procédé, prises seules ou en combinaison :
- le débit déterminé de produit désinfectant est compris entre 5 ml/min et 30 ml/min ;
- le débit du flux d'air d'entraînement est compris entre 1 m³/min et 8 m³/min ;
- on pilote la tête ultrasonique de manière à déterminer une fréquence entre 50 kHz et 100 kHz afin d'obtenir une granulométrie moyenne du brouillard comprise entre 35 micromètres et 55 micromètres.
- la durée de la désinfection est comprise entre 45 min et 180 min,
- le volume du local à désinfecter est compris entre 5 m³ et 250 m³.

Selon un mode de réalisation, le produit désinfectant est choisi parmi (ou comprendre) les produits suivants, le cas échéant en mélange :
- peroxyde d'hydrogène,
- acide acétique,
- acide péracétique.

L'invention concerne encore l'utilisation du dispositif de nébulisation de l'invention pour la désinfection par voie aérienne de locaux.

L'invention concerne également le procédé selon l'invention pour la désinfection par voie aérienne de locaux.

L'invention sera mieux comprise à la lecture de la description suivante accompagnée des figures en annexe parmi lesquelles :

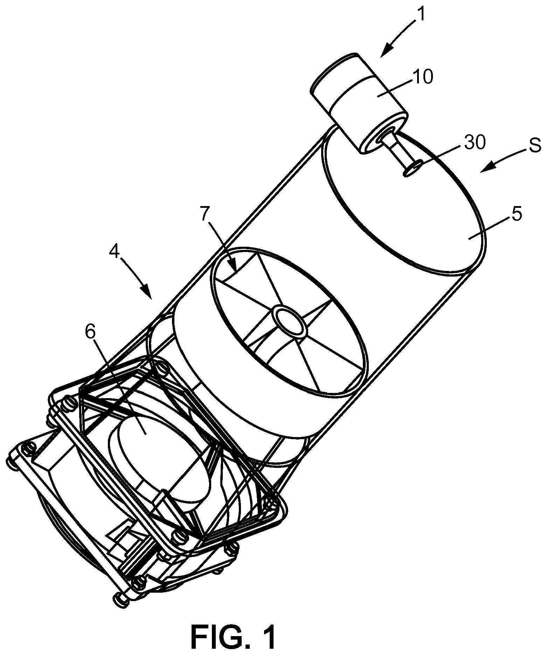

- La figure 1 est une vue en perspective d'un dispositif de nébulisation conforme à l'invention selon un mode de réalisation,
- La figure 2 est une vue en coupe du dispositif de la figure 1, selon un plan vertical passant par l'axe de la tuyère,

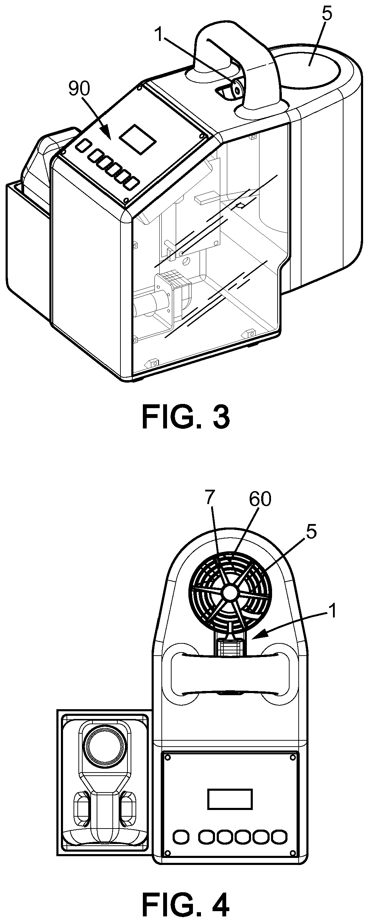

- La figure 3 est une vue en perspective d'un dispositif selon un second mode de réalisation,
- La figure 4 est une vue de dessus du dispositif de la figure 3,
- La figure 5 est une vue en coupe du dispositif de la figure 3, selon un plan vertical passant par la tuyère,
- La figure 6 est une modélisation d'un logiciel de mécanique de fluide numérique simulant le flux annulaire obtenu grâce à un rétrécissement de tuyère en direction d'un orifice d'échappement, un étroit passage annulaire étant ménagé entre le bord dudit orifice et un obstacle coaxial à la tuyère, cette modélisation mettant en évidence d'importantes turbulences en sortie de tuyère.

Aussi l'invention concerne tout d'abord un dispositif de nébulisation pour la désinfection par voie aérienne, comprenant :
- une tête ultrasonique 1 comprenant un transducteur 2 et une sonotrode 3 configurée pour transmettre les vibrations depuis le transducteur 2 vers une surface d'éjection 30 de particules, la sonotrode 3 comportant un canal d'amenée 31 du produit à pulvériser vers la surface d'éjection 30,
- un système aéraulique 4 configuré pour créer un flux d'air d'entraînement des particules pulvérisées par la tête ultrasonique, comprenant une tuyère 5 canalisant le flux d'air jusqu'à une sortie d'air S.

Une telle combinaison de caractéristique est connue en soi des dispositifs FR 2 285 930 A1, EP 2090 187 A1 ou EP 2 090 370 qui reposent sur l'utilisation d'une tête ultrasonique comprenant un transducteur piézoélectrique, et une sonotrode conique, et un système aéraulique configuré pour créer un flux d'air d'entraînement des particules pulvérisées par la tête ultrasonique, l'axe de la tête ultrasonique étant dans ces antériorités sensiblement coaxiale à la tuyère.

Plus encore, et de manière notable dans ces antériorités un rétrécissement de tuyère en direction de son orifice d'échappement forme un étroit passage annulaire ménagé entre le bord dudit orifice et celui du plateau de pulvérisation. Cette section réduite de passage assure un courant d'air annulaire de vitesse d'écoulement très élevée, à proximité immédiate du plateau de pulvérisation, permettant d'entrainer la totalité du brouillard en évitant la formation de filets de liquide sur le plateau, et comme expliqué dans le document FR 2 285 930 A1.

La figure 6 est une modélisation d'un logiciel de mécanique de fluide simulant un tel flux annulaire obtenu grâce à un rétrécissement de tuyère en direction d'un orifice d'échappement, un étroit passage annulaire étant ménagé entre le bord dudit orifice et un obstacle coaxial à la tuyère.

Cette modélisation a pour objet d'illustrer les mouvements du flux d'air en aval de l'orifice d'échappement (selon le sens d'écoulement) tels qu'ils seront observés dans les dispositifs de l'état de la technique connus des documents FR 2 285 930, EP 2 090 187 A1 ou EP 2 090 370 A1 lorsque ce dernier entraîne les particules pulvérisées : l'obstacle coaxial à la tuyère représente ici la tête ultrasonique de ces antériorités. La modélisation met en évidence la présence de turbulences importantes, et en particulier la présence de tourbillons visibles à la figure 6.

L'invention est née de la constatation par l'inventeur que de telles turbulences dans le flux d'air entraînant les particules pulvérisées ne permettent pas de garantir la maîtrise et stabilité du brouillard : ces turbulences ont tendance à provoquer le regroupement entre elles des gouttelettes du brouillard, altérant la granulométrie de la pulvérisation générée par la tête ultrasonique.

Plus encore l'invention est née de la volonté de l'inventeur de réduire sensiblement ces turbulences, et dans le but d'éviter d'altérer autant que possible la granulométrie du brouillard généré par la surface d'éjection 30 de la tête ultrasonique 1. Selon un premier aspect, l'invention concerne ainsi un nouvel agencement entre la tête ultrasonique et la tuyère qui a pour objet de minimiser les turbulences dans le flux d'entrainement en aval de la surface de pulvérisation.

Selon l'invention, la tête ultrasonique 1 est agencée au niveau de la sortie d'air S de manière à positionner dans le flux d'air d'entraînement la surface d'éjection 30 et une partie de longueur de la sonotrode 3 à partir de ladite surface d'éjection 30 qui s'étendant intérieurement au diamètre de l'orifice de sortie de la tuyère, tandis qu'une partie du corps de la tête ultrasonique fait saillie radialement extérieurement au-delà du diamètre de la tuyère 5 et de manière à ne pas être soumis audit flux d'air d'entrainement.

La section du flux d'entrainement selon la direction orthogonale au flux peut être sensiblement un disque. Le transducteur 2 peut se composer d'une ou plusieurs lamelles piézocéramiques, et sont raccordées à une électronique de commande. La sonotrode 3 présente un corps de révolution terminé par un plateau formant ladite surface d'éjection 30, sensiblement perpendiculaire à la surface d'éjection 30. Le corps de la tête ultrasonique 1 peut encore comporter un carter 10, en particulier cylindrique recouvrant le transducteur 2, voire une électronique de commande du transducteur piézoélectrique, ainsi que une partie proximale de la sonotrode 3, la partie distale de la sonotrode portant la surface d'éjection, s'étendant axialement au-delà du carter 10, en particulier axialement au carter cylindrique.

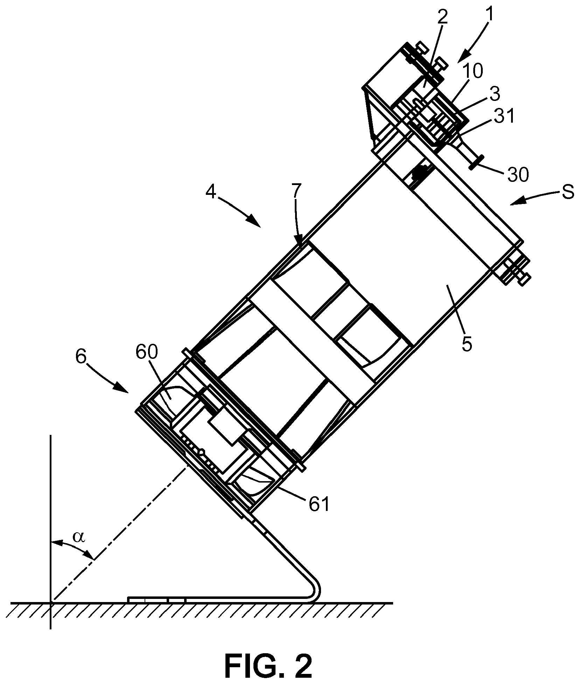

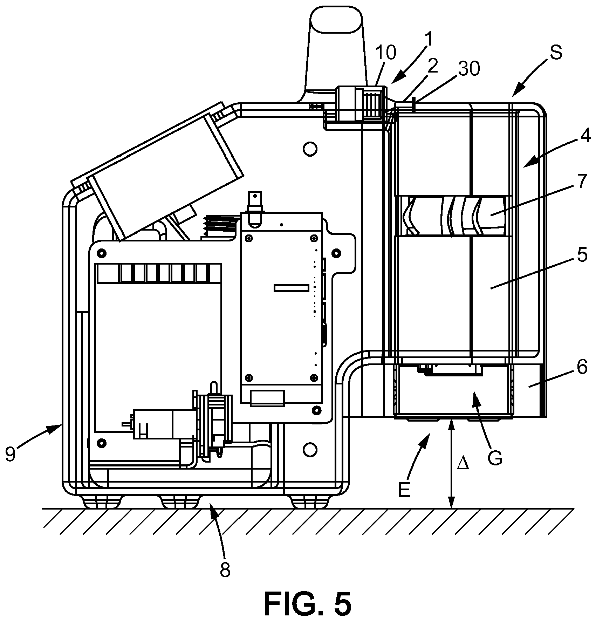

On remarque que l'agencement avantageux de la tête ultrasonique 1 par rapport à la tuyère 5 permet de positionner au moins en partie (voir figure 2), voire totalement (voir figure 5) le carter 10 en saillie radialement au-delà du diamètre de l'orifice de sortie de la tuyère et de manière à ne pas soumettre cette partie au flux d'entrainement : on limite ainsi la dimension de l'obstacle à l'origine des perturbations.

Selon un mode de réalisation minimisant au maximum les turbulences, le carter 10 fait entièrement saillie extérieurement au-delà du diamètre de l'orifice de sortie de la tuyère et de manière à ne pas être soumis audit flux d'air d'entrainement. Avantageusement, on positionne dans le flux d'entrainement seulement la surface d'éjection 30 et la partie distale de la sonotrode 3, de plus petit diamètre, et comme illustré à titre d'exemple à la figure 5.

Selon l'invention, la partie de la tête ultrasonique soumise au flux d'entraînement est positionnée au niveau de la sortie S de la tuyère 5 : on évite ainsi que les particules pulvérisées contenues dans le flux d'air d'entraînement ne se déposent pas sur les parois internes de la tuyère 5.

Selon un mode de réalisation, la partie de la tête ultrasonique 1 soumise au flux d'entraînement peut être ainsi positionnée en aval de l'orifice de sortie de la tuyère 5, selon le sens d'écoulement du flux d'air : on s'assure ainsi que les particules pulvérisées contenues dans le flux d'air d'entraînement ne se déposent pas sur les parois internes de la tuyère 5, et ainsi que la totalité du débit de brouillard généré est entrainée dans l'atmosphère du local à désinfecter. Il est toutefois à noter que cet objectif peut toujours être raisonnablement atteint en positionnant cette partie très légèrement à l'intérieur de la tuyère 5. Selon un mode de réalisation, la partie de la tête ultrasonique soumise au flux d'entraînement peut être positionnée à proximité immédiate de l'orifice de sortie de la tuyère 5.

On remarque que la sonotrode 3 présente un corps de révolution terminé par un plateau formant ladite surface d'éjection 30, l'axe de la sonotrode définissant alors l'axe de la tête ultrasonique. On remarque encore que l'axe de la tête ultrasonique 1 peut être agencé sensiblement perpendiculaire à l'axe de la tuyère 5. On positionne ainsi la surface d'éjection 30 sensiblement parallèle à l'axe de la tuyère 5 et à la direction du flux d'entraînement généré par cette tuyère.

Avantageusement, la sonotrode est de manière classique un corps en métal et qui ne se détériore donc pas au contact des produits employés pour la désinfection par voie aérienne.

Le système aéraulique 4 est configuré pour créer un flux d'air d'entraînement, selon un écoulement laminaire, c'est-à-dire non turbulent. A cet effet, la tuyère 5 est de préférence de section constante en longueur, au moins au niveau de la sortie de tuyère : la tuyère 5 peut ainsi être de section constante sur toute sa hauteur. La tuyère 5 est constituée essentiellement par un corps cylindrique dont la base à l'embouchure inférieure constitue une entrée pour la tuyère, et la base à l'embouchure supérieure ladite sortir d'air S. La vitesse du flux d'air d'entrainement juste en amont de la sortie de la tuyère peut être compris entre 1 m/s et 10 m/s au centre de la tuyère 5.

Le diamètre interne du cylindre peut être compris entre 8 cm et 25 cm. Le débit du flux d'air d'entraînement peut être compris entre 1 m³/min et 8 m³/min. Un tel débit d'air assure lors de la désinfection d'un local un recyclage de l'air du local.

Le système aéraulique peut comprendre un moyen 6 électrique de ventilation disposé en partie inférieure de la tuyère 5, ayant pour fonction de générer le flux d'air. Ce moyen peut être un ventilateur positionné en partie basse de la tuyère dont l'axe de rotation de l'hélice 60 est sensiblement coaxial à l'axe de la tuyère 5. Ce ventilateur peut comporter un carter 61 extérieur, sensiblement cylindrique, s'étendant en prolongement du cylindre de la tuyère 5, sensiblement de même diamètre utile que la tuyère. Le ventilateur électrique peut être un ventilateur d'une puissance modérée, inférieure à 20 Watts, par exemple comprise entre 13 Watts et 19 Watts. La puissance du dispositif de nébulisation dans son ensemble (à savoir pour alimenter le ventilateur, la tête ultrasonique et l'électronique de contrôle) peut être inférieure à 100 W.

Selon un mode de réalisation, un système d'aubage 7 peut être agencé entre ladite sortie d'air S et le moyen électrique de ventilation 6, configuré pour redresser le flux généré par ledit moyen électrique de ventilation 6. Les aubes 70 du système 7 s'étendent respectivement selon des directions radiales à la tuyère. Leur profil courbe des aubes 70 permet de redresser le flux tourbillonnant généré par le rotor du ventilateur électrique en un flux laminaire rectiligne. On remarque encore que ce système d'aubage 7 constitue de préférence le seul obstacle traversé par le flux d'air dans la tuyère 5.

Le dispositif se présente de préférence sous la forme d'un dispositif portatif qui peut être avantageusement de poids modéré inférieur à 10 kg, voire inférieur à 6 kg. Il comporte une surface d'appui 8 permettant de positionner la sortie d'air S de la tuyère 5 vers le haut, lorsque posée sur une surface horizontale.

Selon un mode de réalisation, l'axe de la tuyère 5 peut être incliné par rapport à la verticale suivant un angle α compris entre 0° et 60°, tel que par exemple 45°. Un tel mode de réalisation est illustré à titre d'exemple à la figure 2. Selon un autre mode de réalisation, l'axe de la tuyère peut être sensiblement vertical, et comme illustré à la figure 5.

Dans tous les cas, le flux d'air entraînant les particules pulvérisées est dirigé vers le haut, soit à la verticale, soit incliné par rapport à la verticale du même angle que l'axe de la tuyère. Lorsque la tuyère est verticale, la surface de pulvérisation 31 est de préférence sensiblement verticale et comme illustré à la figure 5. Lorsque la tuyère est inclinée, la surface de pulvérisation 31 est de préférence dirigée vers le bas, et comme illustré à la figure 2.

Le dispositif peut comporter un boîtier 9 recevant intérieurement une électronique de contrôle, ladite surface inférieure dudit boitier 9 constituant ladite surface d'appui 8. Ledit système aéraulique 4 peut être agencé solidaire du boitier, positionné en porte à faux par rapport à la surface d'appui 8 du boitier 9, et de manière à positionner ladite entrée d'air E inférieure du système aéraulique 4 à distance Δ de la surface horizontale. Une interface utilisateur 90 peut être prévue en particulier sur la surface supérieure du boitier. Un compartiment ouvert 91 peut être solidaire du boîtier, latéralement, et destiné à recevoir un réservoir tel qu'un bidon de solution désinfectante.

De manière générale, une pompe du type péristaltique est configurée pour acheminer le produit à pulvériser jusqu'au canal d'amenée 31 de la tête ultrasonique 1 : cette pompe permet de maitriser le débit de produit pulvérisé par la tête ultrasonique 1.

L'invention concerne encore un procédé pour la désinfection par voie aérienne mis en œuvre au moyen d'un dispositif de nébulisation selon l'invention et dans lequel :
- on génère un brouillard désinfectant de débit déterminé par l'action de la surface d'éjection 30 de la tête ultrasonique 1 en alimentant en produit désinfectant le canal d'amenée selon le débit déterminé,
- on entraîne le brouillard désinfectant de débit déterminé dans un flux d'air généré à la sortie de la tuyère 5.

Le brouillard est ainsi généré par la technique des ultrasons grâce à la buse ultrasonique qui assure la qualité de la granulométrie du brouillard : cette buse comprend un transducteur 2 du type piézoélectrique qui est piloté à une fréquence proche de sa fréquence de résonnance de la sonotrode. Le produit liquide à pulvériser est canalisé (de manière continue) dans le conduit d'amenée qui débouche, sur la surface de pulvérisation 30. La fréquence peut être déterminée entre 50 kHz et 100 kHz afin d'obtenir une granulométrie du brouillard de diamètre moyen compris entre 35 micromètres et 55 micromètres, à savoir une granulométrie donnant satisfaction en terme de performance de désinfection. Le débit du produit pulvérisé est maitrisé par l'utilisation d'une pompe (péristaltique) qui alimente la buse ultrasonique selon le débit déterminé.

L'association de cette pompe péristaltique et de la buse ultrasonique 3 permet en soi d'obtenir un brouillard dont la répartition de la granulométrie et le débit sont déterminés selon les caractéristiques de la désinfection souhaitée. Ce brouillard est ensuite entrainé dans le local à traiter par le flux d'air d'entraînement généré par le système aéraulique 4 qui assure également un brassage de l'air du local à désinfecter.

De manière notable, le dispositif de nébulisation est conçu de manière à ne pas détériorer la qualité du brouillard généré par la buse ultrasonique, ni même diminuer la quantité de produit délivré dans la pièce, en particulier grâce au positionnement particulier de la buse ultrasonique 1 par rapport à la tuyère 5, ou encore par le choix d'une section de tuyère droite, constante sur la hauteur de la tuyère.

Selon un mode de réalisation :
- le débit déterminé de produit désinfectant peut être compris entre 5 ml/min et 30 ml/min
- le débit du flux d'air d'entraînement peut être compris entre 1 m³/min et 8 m³/min

La durée de la désinfection est comprise entre 45 min et 180 min. Le dispositif de nébulisation peut éventuellement présenter une horloge permettant de déterminer la durée de la désinfection et un circuit interrompant automatiquement la pulvérisation à l'issue de cette durée.

Le produit désinfectant peut être choisi parmi les produits suivants, ou comprendre les produits suivants, le cas échéant en mélange :
- peroxyde d'hydrogène,
- acide acétique,
- acide péracétique.

Le dispositif de nébulisation et le procédé de désinfection conformes à l'invention trouvent une application pour la désinfection de local, en particulier de 5 m³ à 250 m³, ou encore de compartiments de véhicule.

### NOMENCLATURE

- 1.: Tête ultrasonique
- 2.: Transducteur,
- 3.: Sonotrode,
- 30.: Surface d'éjection,
- 31.: Canal d'amenée,
- 4.: Système aéraulique,
- 5.: Tuyère,
- 6.: Moyens de ventilation électrique,
- 60.: Hélice (ventilateur),
- 7.: Système d'aubage (le redressement du flux du ventilateur),
- 70.: Aubes,
- 9.: Boiter,
- 90.: Interface utilisateur,
- 91.: Compartiment (Bidon)
- 10.: Carter tête ultrasonique

## Revendications

1. Dispositif de nébulisation d'un produit convenant pour la désinfection par voie aérienne, comprenant :
- une tête ultrasonique (1) comprenant un transducteur (2) et une sonotrode (3) configurée pour transmettre les vibrations depuis le transducteur (2) vers une surface d'éjection (30) de particules, la sonotrode (3) comportant un canal d'amenée (31) du produit à pulvériser vers la surface d'éjection (30), la sonotrode (3) présentant un corps de révolution terminé par un plateau formant ladite surface d'éjection (30), l'axe de la sonotrode définissant l'axe de la tête ultrasonique,
- un système aéraulique (4) configuré pour créer un flux d'air d'entraînement des particules pulvérisées par la tête ultrasonique, comprenant une tuyère (5) canalisant le flux d'air jusqu'à une sortie d'air (S),
dans lequel la tête ultrasonique (1) est agencée au niveau de la sortie d'air (S) de manière à
positionner dans le flux d'air d'entraînement la surface d'éjection (30) et une partie de longueur de la sonotrode (3) à partir de ladite surface d'éjection (30) tandis qu'une partie du corps de la tête ultrasonique fait saillie radialement extérieurement au-delà du diamètre de l'orifice de sortie d'air (S) de la tuyère (5) et de manière à ne pas être soumis audit flux d'air d'entraînement,
et dans lequel l'axe de la tête ultrasonique (1) est agencé sensiblement perpendiculaire à l'axe de la tuyère (5), la surface d'éjection (30) étant sensiblement parallèle à l'axe de la tuyère (5).

2. Dispositif selon la revendication 1, dans lequel la tête ultrasonique (1) présente un carter (10), en particulier cylindrique, recouvrant le transducteur (2), voire une électronique de commande du transducteur piézoélectrique, ainsi qu'une partie proximale de la sonotrode (3), une partie distale de la sonotrode portant la surface d'éjection (10) s'étendant axialement au-delà du carter (10) et dans lequel la tête ultrasonique (1) est agencée de manière à positionner dans le flux d' entraînement seulement la surface d'éjection (30) et la partie distale de la sonotrode (3) et de sorte que le carter (10) soit positionné extérieurement en saillie au-delà du diamètre de l'orifice de sortie d'air (S) pour ne pas être soumis audit flux d'air d' entraînement.

3. Dispositif selon l'une des revendications 1 à 2, dans lequel la partie de la tête ultrasonique soumise au flux d'entraînement est positionnée en aval de l'orifice de sortie d'air (S) de la tuyère (5), selon le sens d'écoulement du flux d'air, et de préférence la partie de la tête ultrasonique soumise au flux d'entraînement est positionnée à proximité immédiate de l'orifice de sortie d'air (S) de la tuyère (5).

4. Dispositif selon l'une des revendications 1 à 3, dans lequel la tuyère (5) est de section constante en longueur, au moins au niveau de la sortie d'air (S) de tuyère (5), de préférence la tuyère (5) est de section constante sur toute sa hauteur, de façon plus préférée la tuyère (5) est constituée essentiellement par un corps cylindrique dont la base à l'embouchure inférieure constitue une entrée pour la tuyère, et la base à l'embouchure supérieure ladite sortie d'air (S) lorsque la sortie d'air (S) de la tuyère (5) est positionnée vers le haut.

5. Dispositif selon l'une des revendications 1 à 4, dans lequel ledit système aéraulique (4) comprend un moyen électrique de ventilation (6), disposé en partie inférieure de la tuyère (5), lorsque la sortie d'air (S) de la tuyère (5) est positionnée vers le haut, de préférence ledit moyen de ventilation électrique (6) est un ventilateur positionné en partie basse de la tuyère (5) dont l'axe de rotation de l'hélice (60) est sensiblement coaxial à l'axe de la tuyère (5).

6. Dispositif selon la revendication 5, comprenant un système d'aubage (7) agencé entre ladite sortie d'air (S) et le moyen électrique de ventilation (6), configuré pour redresser le flux généré par ledit moyen électrique de ventilation (6).

7. Dispositif selon l'une des revendications 1 à 6 comprenant une pompe péristaltique configurée pour acheminer le produit à pulvériser jusqu'au canal d'amenée (31) de la tête ultrasonique (1).

8. Dispositif selon l'une des revendications 1 à 7 portatif, comportant une surface d'appui (8) permettant de positionner la sortie d'air (S) de la tuyère (5) vers le haut, lorsque posée sur une surface horizontale, de préférence dans lequel l'axe de la tuyère (5) est sensiblement à la verticale, ou suivant une direction inclinée par rapport à la verticale d'un angle α compris entre 0° et 60°, tel que par exemple 45° lorsque la surface d'appui (8) repose sur la surface horizontale, de façon plus préférée comprenant un boîtier (9) recevant intérieurement une électronique de contrôle, ladite surface inférieure dudit boitier (9) constituant ladite surface d'appui (8), ledit système aéraulique (4) étant agencé solidaire du boitier, positionné en porte à faux par rapport à la surface d'appui (8) du boitier (9), de manière à positionner ladite entrée d'air (E) inférieure du système aéraulique (4) à distance (Δ) de la surface horizontale.

9. Procédé pour la désinfection par voie aérienne mis en œuvre au moyen d'un dispositif de nébulisation selon l'une des revendications 1 à 8, dans lequel :
- on génère un brouillard désinfectant de débit déterminé par l'action de la surface d'éjection de la tête ultrasonique (1) en alimentant en produit désinfectant le canal d'amenée selon le débit déterminé,
- on entraîne le brouillard désinfectant de débit déterminé dans le flux d'air généré à la sortie d'air (S) de la tuyère.

10. Procédé selon la revendication 9, dans lequel le débit déterminé de produit désinfectant est compris entre 5 ml/min et 30 ml/min ou dans lequel le débit du flux d'air d'entraînement est compris entre 1 m³/min et 8 m³/min.

11. Procédé selon l'une des revendications 9 à 10, dans lequel on pilote la tête ultrasonique de manière à déterminer une fréquence entre 50 kHz et 100 kHz afin d'obtenir une granulométrie moyenne du brouillard comprise entre 35 micromètres et 55 micromètres.

12. Procédé selon l'une des revendications 9 à 11, dans lequel la durée de la pulvérisation est comprise entre 45 min et 180 min.

13. Procédé selon l'une des revendications 9 à 12, dans lequel le produit désinfectant est choisi parmi les produits suivants ou comprend les produis suivants, le cas échéant en mélange :
- peroxyde d'hydrogène,
- acide péracétique,
- acide acétique.

14. Utilisation du dispositif de nébulisation selon l'une quelconque des revendications 1 à 8 pour la désinfection par voie aérienne de locaux.

## Patentansprüche

1. Zerstäuber-Vorrichtung für ein Produkt, das sich zur Desinfektion über den Luftweg eignet, umfassend:
- einen Ultraschallkopf (1), der einen Wandler (2) und eine Sonotrode (3) umfasst, die dazu konfiguriert ist, die Schwingungen vom Wandler (2) auf eine Ausstoßfläche (30) für Partikel zu übertragen, wobei die Sonotrode (3) einen Zufuhrkanal (31) zur Ausstoßfläche (30) für das zu versprühende Produkt umfasst, wobei die Sonotrode (3) einen Rotationskörper aufweist, der in einer Platte endet, welche die Ausstoßfläche (30) bildet, wobei die Achse der Sonotrode die Achse des Ultraschallkopfes definiert,
- ein Luftsystem (4), das dazu konfiguriert ist, einen Antriebsluftstrom für die vom Ultraschallkopf versprühten Partikel zu erzeugen, umfassend eine Düse (5), die den Luftstrom zu einem Luftauslass (S) leitet;
wobei der Ultraschallkopf (1) auf Höhe des Luftauslasses (S) angeordnet ist, so dass die Ausstoßfläche (30) und ein Teil der Länge der Sonotrode (3) ausgehend von der Ausstoßfläche (30) im Antriebsluftstrom positioniert werden, während ein Teil des Körpers des Ultraschallkopfes radial nach außen über den Durchmesser der Öffnung des Luftauslasses (S) der Düse (5) hinausragt, und zwar so, dass er nicht dem Antriebsluftstrom ausgesetzt ist,
und wobei die Achse des Ultraschallkopfes (1) im Wesentlichen senkrecht zur Achse der Düse (5) angeordnet ist, wobei die Ausstoßfläche (30) im Wesentlichen parallel zur Achse der Düse (5) verläuft.

2. Vorrichtung nach Anspruch 1, wobei der Ultraschallkopf (1) ein insbesondere zylindrisches Gehäuse (10) aufweist, das den Wandler (2) abdeckt, sowie eine Elektronik zur Steuerung/Regelung des piezoelektrischen Wandlers und einen proximalen Teil der Sonotrode (3), wobei ein distaler Teil der Sonotrode, der die Ausstoßfläche (10) trägt, sich axial über das Gehäuse (10) hinaus erstreckt, und wobei der Ultraschallkopf (1) so angeordnet ist, dass nur die Ausstoßfläche (30) und der distale Teil der Sonotrode (3) im Antriebsstrom positioniert sind, und so, dass das Gehäuse (10) nach außen über den Durchmesser der Öffnung des Luftauslasses (S) hinausragt, um dem Antriebsluftstrom nicht ausgesetzt zu sein.

3. Vorrichtung nach einem der Ansprüche 1 bis 2, wobei der Teil des Ultraschallkopfes, der dem Antriebsstrom ausgesetzt ist, in Strömungsrichtung des Luftstroms stromabwärts der Öffnung des Luftauslasses (S) der Düse (5) positioniert ist, und wobei vorzugsweise der Teil des Ultraschallkopfes, der dem Antriebsstrom ausgesetzt ist, in unmittelbarer Nähe der Öffnung des Luftauslasses (S) der Düse (5) positioniert ist.

4. Vorrichtung nach einem der Ansprüche 1 bis 3, wobei die Düse (5) zumindest auf Höhe des Luftauslasses (S) der Düse (5) einen konstanten Querschnitt aufweist, wobei die Düse (5) bevorzugt über ihre gesamte Höhe einen konstanten Querschnitt aufweist, wobei die Düse (5) noch mehr bevorzugt im Wesentlichen aus einem zylindrischen Körper besteht, dessen Basis an der unteren Mündung einen Einlass für die Düse darstellt, und die Basis an der oberen Mündung den Luftauslass (S), wenn der Luftauslass (S) der Düse (5) nach oben zeigt.

5. Vorrichtung nach einem der Ansprüche 1 bis 4, wobei das Luftsystem (4) eine elektrische Belüftungseinrichtung (6) umfasst, die in dem unteren Teil der Düse (5) angeordnet ist, wenn der Luftauslass (S) der Düse (5) nach oben zeigt, wobei die elektrische Belüftungseinrichtung (6) bevorzugt ein Ventilator ist, der in dem unteren Teil der Düse (5) positioniert ist, wobei die Drehachse der Luftschraube (60) davon im Wesentlichen koaxial zur Achse der Düse (5) verläuft.

6. Vorrichtung nach Anspruch 5, umfassend ein Schaufelsystem (7), das zwischen dem Luftauslass (S) und der elektrischen Belüftungseinrichtung (6) angeordnet und dazu konfiguriert ist, die durch die elektrische Belüftungseinrichtung (6) erzeugte Strömung zu korrigieren.

7. Vorrichtung nach einem der Ansprüche 1 bis 6, umfassend eine Peristaltik-Pumpe, die dazu konfiguriert ist, das zu versprühende Produkt zum Zufuhrkanal (31) des Ultraschallkopfes (1) zu befördern.

8. Vorrichtung nach einem der Ansprüche 1 bis 7, welche tragbar ist, umfassend eine Auflagefläche (8), die es erlaubt, den Luftauslass (S) der Düse (5) so zu positionieren, dass er nach oben zeigt, wenn sie auf einer horizontalen Fläche angeordnet ist, wobei bevorzugt die Achse der Düse (5) im Wesentlichen vertikal ist oder einer relativ zur Vertikalen geneigten Richtung folgt, unter einem Winkel α zwischen 0° und 60°, beispielsweise 45°, wenn die Auflagefläche (8) auf der horizontalen Fläche aufliegt, noch mehr bevorzugt umfassend eine Aufnahme (9), die intern eine Steuer-/Regelelektronik aufnimmt, wobei die Unterseite der Aufnahme (9) die Auflagefläche (8) bildet, wobei das Luftssystem (4) fest mit der Aufnahme verbunden angeordnet ist, relativ zur Auflagefläche (8) der Aufnahme (9) freitragend positioniert, um den unteren Lufteinlass (E) des Luftsystems (4) in einem Abstand (Δ) von der horizontalen Fläche zu positionieren.

9. Verfahren zur Desinfektion über den Luftweg, das mittels einer Zerstäuber-Vorrichtung gemäß einem der Ansprüche 1 bis 8 durchgeführt wird, wobei:
- ein Desinfektionsnebel mit einer Durchflussrate erzeugt wird, die durch die Wirkung der Ausstoßfläche des Ultraschallkopfes (1) bestimmt wird, indem dem Zufuhrkanal gemäß der bestimmten Durchflussrate ein Desinfektionsmittel zugeführt wird,
- der Desinfektionsnebel mit der bestimmten Durchflussrate in dem am Luftauslass (S) der Düse erzeugten Luftstrom angetrieben wird.

10. Verfahren nach Anspruch 9, wobei die bestimmte Durchflussrate des Desinfektionsmittels zwischen 5 ml/min und 30 ml/min liegt, oder wobei die Durchflussrate des Antriebsluftstroms zwischen 1 m³/min und 8 m³/min liegt.

11. Verfahren nach einem der Ansprüche 9 bis 10, wobei der Ultraschallkopf so gesteuert wird, dass eine Frequenz zwischen 50 kHz und 100 kHz bestimmt wird, um eine durchschnittliche Teilchengröße des Nebels zwischen 35 Mikrometern und 55 Mikrometern zu erhalten.

12. Verfahren nach einem der Ansprüche 9 bis 11, wobei die Sprühdauer zwischen 45 min und 180 min liegt.

13. Verfahren nach einem der Ansprüche 9 bis 12, wobei das Desinfektionsmittel aus den folgenden Produkten ausgewählt ist oder gegebenenfalls die folgenden Produkte in einer Mischung umfasst:
- Wasserstoffperoxid,
- Peressigsäure,
- Essigsäure.

14. Verwendung der Zerstäuber-Vorrichtung gemäß einem der Ansprüche 1 bis 8 zur Desinfektion von Räumlichkeiten über den Luftweg.

## Claims

1. Device for atomising a product suitable for air way disinfection, comprising:
- an ultrasound probe (1) comprising a transducer (2) and a sonotrode (3) configured to transmit the vibrations from the transducer (2) to a particle ejection surface (30), the sonotrode (3) comprising an intake channel (31) of the product to be sprayed towards the ejection surface (30), the sonotrode (3) having a body of revolution terminated by a plate forming said ejection surface (30), with the axis of the sonotrode defining the axis of the ultrasound probe,
- an aeraulic system (4) configured to create a flow of air for driving particles sprayed by the ultrasonic probe, comprising a nozzle (5) that channels the flow of air to an air outlet (S),
wherein the ultrasonic probe (1) is arranged on the air outlet (S) in such a way as to position in the flow of air for driving the ejection surface (30) and a portion of the length of the sonotrode (3) starting from said ejection surface (30) while a portion of the body of the ultrasonic probe protrudes radially exteriorly beyond the diameter of the air outlet (S) orifice of the nozzle (5) and in such a way as to not be subjected to said flow of air for driving, and wherein the axis of the ultrasound probe (1) is arranged substantially perpendicular to the axis of the nozzle (5), the ejection surface (30) being substantially parallel to the axis of the nozzle (5).

2. Device according to claim 1, wherein the ultrasonic probe (1) has a casing (10), in particular cylindrical, that covers the transducer (2), even control electronics of the piezoelectric transducer, as well as a proximal portion of the sonotrode (3), with a distal portion of the sonotrode supporting the ejection surface (10) extending axially beyond the casing (10) and wherein the ultrasonic probe (1) is arranged in such a way as to position in the flow for driving only the ejection surface (30) and the distal portion of the sonotrode (3) and in such a way that the casing (10) is positioned exteriorly protruding beyond the diameter of the orifice of the air outlet in order to not be subjected to said flow of air for driving.

3. Device according to claim 1 or 2, wherein the portion of the ultrasonic probe subjected to the flow for driving is positioned downstream of the air outlet (S) orifice of the nozzle (5), according to the direction of flow of the flow of air, and preferably the portion of the ultrasonic probe subjected to the flow for driving is positioned in the immediate vicinity of the air outlet (S) orifice of the nozzle (5).

4. Device according to one of claims 1 to 3, wherein the nozzle (5) is of a constant section in length, at least at the air outlet (S) of the nozzle (5), preferably the nozzle (5) is of a constant section over the entire height thereof, more preferably the nozzle (5) is substantially constituted of a cylindrical body of which the base at the lower mouth forms an inlet for the nozzle, and the base at the upper mouth said air outlet (S) when the air outlet (S) of the nozzle (5) is positioned upwards.

5. Device according to one of claims 1 to 4, wherein said aeraulic system (4) comprises an electrical means of ventilation (6), arranged in the lower portion of the nozzle (5), when the air outlet (S) of the nozzle (5) is positioned upwards, preferably said electrical means of ventilation (6) is a fan positioned in the lower portion of the nozzle (5) of which the axis of rotation of the propeller (60) is substantially coaxial to the axis of the nozzle (5).

6. Device according to claim 5, comprising a vane system (7) arranged between said air outlet (S) and the electrical means of ventilation (6), configured to correct the flow generated by said electrical means of ventilation (6).

7. Device according to one of claims 1 to 6 comprising a peristaltic pump configured to convey the product to be sprayed to the intake channel (31) of the ultrasonic probe (1).

8. Device according to one of claims 1 to 7 that is portable, comprising a support surface (8) that makes it possible to position the air outlet (S) of the nozzle (5) upwards, when placed on a horizontal surface, preferably wherein the axis of the nozzle (5) is substantially at the vertical, or according to a direction inclined in relation to the vertical by an angle α between 0° and 60°, such as for example 45° when the support surface (8) is resting on a horizontal surface, more preferably comprising a case (9) interiorly receiving control electronics, said lower surface of said case (9) constituting said support surface (8), said aeraulic system (4) being arranged integral with the case, positioned overhanging in relation to the support surface (8) of the case (9), in such a way as to position said lower inlet of air (E) of the aeraulic system (4) at a distance (Δ) from the horizontal surface.

9. Method for the air way disinfection implemented by means of a device for atomising according to one of claims 1 to 8, wherein:
- a disinfecting mist of a determined flow rate is generated by the action of the ejection surface of the ultrasonic probe (1) by supplying with disinfecting product the intake channel according to the determined flow rate,
- the disinfecting mist of a determined flow rate is driven in the flow of air generated at the air outlet (S) of the nozzle.

10. Method according to claim 9, wherein the determined flow rate of the disinfecting product is between 5 ml/min and 30 ml/min or wherein the flow rate of the flow of air for driving is between 1 m³/min and 8 m³/min.

11. Method according to one of claims 9 to 10, wherein the ultrasonic probe is controlled so as to determine a frequency between 50 kHz and 100 kHz in order to obtain an average granulometry of the mist between 35 micrometres and 55 micrometres.

12. Method according to one of claims 9 to 11, wherein the duration of the spraying is between 45 min and 180 min.

13. Method according to one of claims 9 to 12, wherein the disinfecting product is chosen from the following products or comprises the following products, where applicable in a mixture:
- hydrogen peroxide,
- peracetic acid,
- acetic acid.

14. Use of the device for atomising according to any of claims 1 to 8 for air way disinfection of premises.
